# EUROPEAN PATENT APPLICATION

(11) **EP 4 717 770 A1**
(43) Date of publication of application: **01.04.2026**
(21) Application number: 23937902.7
(22) Date of filing: 23.05.2023
(51) Int. Cl.: C12N 15/113, C12N 15/864, A61K 35/76, A61P 27/16

(54) **COCHLEAR HAIR CELL SPECIFIC PROMOTER AND USE THEREOF**

(71) Applicant: Otovia Therapeutics, Suzhou, Jiangsu 215000 (CN)
(72) Inventor: CHAI, Renjie, Nanjing, Jiangsu 210024 (CN); QI, Jieyu, Nanjing, Jiangsu 210024 (CN); TAN, Fangzhi, Shanghai 201210 (CN); ZHANG, Liyan, Nanjing, Jiangsu 210024 (CN); JIANG, Lulu, Shanghai 201210 (CN); ZHANG, Shanzhong, Shanghai 201210 (CN); TAN, Chang, Shanghai 201210 (CN); LU, Ling, Nanjing, Jiangsu 210008 (CN); CUI, Zhiping, Shanghai 201210 (CN); SONG, Huaien, Shanghai 201210 (CN); SUN, Sijie, Shanghai 201210 (CN)
(74) Representative: Nederlandsch Octrooibureau
(86) International application number: PCT/CN2023/095739
(87) International publication number: WO 2024/239226

(57) **Abstract**

The present invention relates to a cochlear hair cell specific promoter and the use thereof. The present invention particularly relates to a nucleic acid promoter (SEQ ID NO: 1), the nucleic acid promoter comprising an enhancer sequence (SEQ ID NO: 2) and a core promoter element (SEQ ID NO: 3), wherein the enhancer sequence has a sequence shown as SEQ ID NO: 2 or a sequence having at least 90% sequence identity with same, and the core promoter element has a sequence shown as SEQ ID NO: 3 or a sequence having at least 90% sequence identity with same. The promoter (SEQ ID NO: 1) can specifically guide gene expression in cochlear hair cells.

## Description

### FIELD OF THE DISCLOSURE

The invention relates to gene engineering technology, in particular to a method for gene therapy of cochlear hair cells.

### BACKGROUND OF THE DISCLOSURE

Deafness is the most common sensory disorder in humans. According to the World Health Organization, deafness affects about 466 million people, including 34 million children, accounting for 6% of the world's population. Among them, sensorineural hearing loss (SNHL) accounts for about 63% of deaf patients. Sensorineural hearing loss is caused by organic damage to the inner ear hair cells and auditory nerves, resulting in impaired sound wave perception, which in turn causes hearing loss. There are many causes of sensorineural hearing loss, including ototoxic drugs, noise, genetics and aging. The cause of congenital sensorineural hearing loss may be environmental factors (such as deafness caused by congenital cytomegalovirus and ototoxic antibiotics) or genetic factors. One in every 500 babies suffers from hereditary hearing loss, and 119 genes related to hereditary hearing loss have been identified. Non-syndromic deafness accounts for 60-70% of hereditary hearing impairment. Currently, more than 67 pathogenic genes for non-syndromic deafness have been identified, and the number of assigned non-syndromic deafness loci has reached 130. Among these 67 non-syndromic deafness genes, 38 are autosomal recessive genes, 25 are autosomal dominant genes, there are 2 X-chromosome-linked non-syndromic deafness genes, and the others are pathogenic genes for mitochondrial and microRNA-related deafness.

As sensory cells, the main function of cochlear hair cells is to convert the mechanical vibration signals of sound into bioelectric signals. Currently, the treatment of deafness is mainly based on instrumental assistance. There are no other effective means except wearing hearing aids and cochlear implants. However, both hearing aids and artificial hearing implants are only alternative treatments with limited effectiveness, and their functions cannot be compared with normal hearing. There is still a lack of ideal treatments for sensorineural hearing loss.

Gene therapy refers to intervening in the occurrence and development of diseases by manipulating genetic material, and achieving the purpose of treating diseases by replacing and correcting abnormal pathogenic genes. In recent years, with the deepening of research on genetic genes and the pathogenesis of deafness, and the establishment of various animal models of deafness, more and more scholars have tried to prevent and treat sensorineural hearing loss through gene therapy. In recent years, gene therapy has shown great potential in the fields of protection and regeneration of auditory nerves and hair cells, and treatment of hereditary deafness, and is expected to become an effective method to solve the problem of sensorineural deafness. Therefore, it is very urgent for people to develop and apply gene transfer technology to restore normal cochlear function.

In recent years, great breakthroughs have been made in gene therapy research for hereditary deafness. Many studies have reversed the hearing phenotype of hereditary deafness mouse models through gene therapy. The mice have successfully retained some hearing by introducing the corresponding wild-type genes into the cochlea, including the mice with GJB2, VGLUT3, TMC, and KCNQ1 gene knockout. This series of research results indicates that gene therapy is expected to become an effective method for treating hereditary deafness. Although great progress has been made in basic research on gene therapy for sensorineural deafness, there remains a long way to go to achieve clinical transformation. At present, one of the main obstacles restricting the clinical transformation of gene therapy for deafness is that genes cannot be stably and continuously expressed for a long time after being introduced into cochlear hair cells, and the transfection efficiency is low and lacks targeting. How to improve the transfection efficiency and targeting of genes introduced into cochlear hair cells is a hot topic for future research. Different promoters exhibit huge differences in expression levels in the same cell type, while specific promoters can specifically express reporter genes in different cell types. Currently, the non-specific ubiquitous CMV promoter derived from human cytomegalovirus and the CAG promoter composed of the CMV enhancer and the chicken β-actin promoter are commonly used for gene therapy. It has been reported that the transgene expression of CMV decreases over time due to transcriptional silencing and lacks targeting. In order to achieve stable and relatively high specific expression in cochlear hair cells, it is necessary to design a specific promoter for deafness gene therapy.

### SUMMARY OF THE DISCLOSURE

In view of the shortcomings of the prior art, the inventors developed a specific promoter Otov-HC (SEQ ID NO: 1), which can specifically promote the expression of transgenes in cochlear hair cells.

The present invention first provides a nucleic acid promoter Otov-HC (SEQ ID NO: 1), which can specifically guide gene expression in cochlear hair cells as a promoter, comprising: (1) an enhancer sequence, and (2) a core promoter element, wherein the enhancer sequence comprises a sequence shown in SEQ ID NO: 2 or a homologous sequence having at least 90% sequence identity thereto and retaining the function of SEQ ID NO: 2. The homologous sequence is a sequence of mammals, preferably primates, and more preferably hominids.

In one or more embodiments, the core promoter element described in (2) is the core promoter element suitable for promoting gene expression in mammalian cells.

In one or more embodiments, the core promoter element described in (2) is the core promoter element of the SHH gene.

In one or more embodiments, the core promoter element described in (2) comprises the sequence shown in SEQ ID NO: 3 or a homologous sequence having at least 90% sequence identity thereto. The homologous sequence is a sequence of mammals, preferably primates, and more preferably hominids.The homologous sequence is the sequence of the core promoter element of the SHH gene of mammals, preferably the SHH gene of primates, more preferably the SHH gene of hominids.

In one or more embodiments, the enhancer sequence described in (1) is located upstream or downstream of the core promoter element described in (2).

In one or more embodiments, the distance between the enhancer sequence described in (1) and the core promoter element described in (2) is 0-5 kb, preferably 0-5 kb, 0-3 kb, 0-2 kb, 0-1 kb, 0-500 bp, and more preferably 0-200 bp.

In one or more embodiments, the enhancer sequence is located 0-500 bp upstream of the core promoter element.

In one or more embodiments, the promoter comprises the sequence shown in SEQ ID NO: 1, or a sequence having at least 90% sequence identity thereto.

The present invention also provides a nucleic acid construct comprising the promoter described in any embodiment of the present invention.

In one or more embodiments, the nucleic acid construct is an expression cassette. The expression cassette comprises the promoter, the multiple cloning site and the terminator from upstream to downstream (5' to 3'). Preferably, the terminator is a polyA terminator.

In one or more embodiments, the nucleic acid construct is a vector, such as a cloning vector, an integration vector, or an expression vector, preferably pAAV.

In one or more embodiments, the nucleic acid construct further comprises a gene of interest, which is operably linked to the promoter.

In one or more embodiments, the gene is selected from: a deafness gene, a tool gene for manipulating genes of cochlear hair cells or expressed in cochlear hair cells.

In one or more embodiments, the deafness gene is a deafness gene expressed in cochlear hair cells.

In one or more embodiments, the deafness gene is a causative gene for non-syndromic deafness.

In one or more embodiments, the deafness gene includes one or more selected from the group consisting of: ATOH1, VGLUT3, TMC1/2, KCNQ1, MLRB3, LHFPL5, OTOF, STRC, MYO7A, CHD7, TMPRSS3, POU3F4, ESPN, SYNE4, DIAPH1, KCNQ4, CEACAM16, MYO6, P2RX2, SMAC, TBC1D24, CD164, OSBPL2, HOMER2, MYO3A, PDE1C, SCD5, USH1C, RDX, DCD C2, LOXHD1, TPRN, SERPINB6, SiX1, CDH23, WHRN, OtoGL, WFS1, MYH9, REST, MCM2, DMXL2, TMIE, PCDH15, GRXC R1, TRIOBP, ILDR1, CIB2, GPSM2, ELMOD3, CABP2, TMEM132E, GRXCR2, EPS8, CLIC5, TRIOBP, ILDR1, SLC26A5.

The present invention also provides an AAV vector system, comprising the nucleic acid construct of any embodiment of the present invention as pAAV and a nucleic acid construct encoding genes required by the AAV virus.

In one or more embodiments, the genes required for AAV virus packaging include one or more selected from the group consisting of: Rep, Cap, E2A, E4, and VA genes.

In one or more embodiments, the AAV vector system comprises an AAV-ie plasmid, a pHelper plasmid, and the pAAV.

The present invention also provides an AAV virus particle comprising the promoter described in any embodiment of the present invention.

The present invention also provides a host cell, comprising or having integrated into its genome: the promoter, nucleic acid construct or AAV vector system described in any embodiment of the present invention.

Preferably, the cells are cochlear hair cells.

The present invention also provides a pharmaceutical composition comprising a pharmaceutically acceptable excipient and one or more selected from the group consisting of: a promoter, a nucleic acid construct, an AAV vector system, an AAV virus particle, and a host cell as described in any embodiment of the present invention.

The present invention also provides an in vitro method for expressing genes in cochlear hair cells, comprising the step of incubating the host cell according to any embodiment of the present invention under conditions suitable for gene expression, wherein the host cell is a cochlear hair cell.

In one or more embodiments, the method further comprises the step of introducing a nucleic acid construct comprising the gene into cochlear hair cells.

In one or more embodiments, the expression of the gene is directed by the promoter. The promoter is in the same expression frame as the gene and is located upstream of the gene.

In one or more embodiments, the gene is selected from: a deafness gene, and various tool genes for manipulating genes of cochlear hair cells or expressed in cochlear hair cells.

In one or more embodiments, the deafness gene is a deafness gene expressed in cochlear hair cells.

In one or more embodiments, the deafness gene is a causative gene for deafness.

In one or more embodiments, the deafness gene includes one or more selected from the group consisting of: ATOH1, VGLUT3, TMC1/2, KCNQ1, MLRB3, LHFPL5, OTOF, STRC, MYO7A, CHD7, TMPRSS3, POU3F4, ESPN, SYNE4, DIAPH1, KCNQ4, CEACAM16, MYO6, P2RX2, SMAC, TBC1D24, CD164, OSBPL2, HOMER2, MYO3A, PDE1C, SCD5, USH1C, RDX, DCD C2, LOXHD1, TPRN, SERPINB6, SiX1, CDH23, WHRN, OtoGL, WFS1, MYH9, REST, MCM2, DMXL2, TMIE, PCDH15, GRXC R1, TRIOBP, ILDR1, CIB2, GPSM2, ELMOD3, CABP2, TMEM132E, GRXCR2, EPS8, CLIC5, TRIOBP, ILDR1, SLC26A5.

The present invention also provides use of the promoter described in any embodiment of the present invention in the preparation of a drug for treating a disease caused by a gene defect in cochlear hair cells, wherein the drug is used to express the gene in cochlear hair cells.

In one or more embodiments, the gene is selected from: a deafness gene, and various tool genes for manipulating genes of cochlear hair cells or expressed in cochlear hair cells.

In one or more embodiments, the deafness gene is a deafness gene expressed in cochlear hair cells.

In one or more embodiments, the deafness gene is a causative gene for deafness.

In one or more embodiments, the deafness gene includes one or more selected from the group consisting of: ATOH1, VGLUT3, TMC1/2, KCNQ1, MLRB3, LHFPL5, OTOF, STRC, MYO7A, CHD7, TMPRSS3, POU3F4, ESPN, SYNE4, DIAPH1, KCNQ4, CEACAM16, MYO6, P2RX2, SMAC, TBC1D24, CD164, OSBPL2, HOMER2, MYO3A, PDE1C, SCD5, USH1C, RDX, DCD C2, LOXHD1, TPRN, SERPINB6, SiX1, CDH23, WHRN, OtoGL, WFS1, MYH9, REST, MCM2, DMXL2, TMIE, PCDH15, GRXC R1, TRIOBP, ILDR1, CIB2, GPSM2, ELMOD3, CABP2, TMEM132E, GRXCR2, EPS8, CLIC5, TRIOBP, ILDR1, SLC26A5.

In one or more embodiments, the disease is hereditary deafness caused by a gene defect in the cochlear hair cells. Preferably, the disease is sensorineural hearing loss. In one or more embodiments, the sensorineural hearing loss includes congenital sensorineural deafness. In one or more embodiments, the congenital sensorineural deafness is congenital hereditary sensorineural deafness.

The present invention also provides a method for treating a disease caused by a gene defect in cochlear hair cells, comprising the step of expressing the gene in the cochlear hair cells of a subject, wherein the gene is operably linked to the promoter described in any embodiment of the present invention.

In one or more embodiments, the method includes the step of introducing a nucleic acid construct or vector comprising the coding sequence of the gene operably linked to the promoter into the cochlear hair cells .

In one or more embodiments, the expression of the gene is directed by the promoter. The promoter is in the same expression frame as the gene and is located upstream of the gene.

In one or more embodiments, the deafness gene is a deafness gene expressed in cochlear hair cells.

In one or more embodiments, the deafness gene is a causative gene for non-syndromic deafness.

In one or more embodiments, the deafness gene includes one or more selected from the group consisting of: ATOH1, VGLUT3, TMC1/2, KCNQ1, MLRB3, LHFPL5, OTOF, STRC, MYO7A, CHD7, TMPRSS3, POU3F4, ESPN, SYNE4, DIAPH1, KCNQ4, CEACAM16, MYO6, P2RX2, SMAC, TBC1D24, CD164, OSBPL2, HOMER2, MYO3A, PDE1C, SCD5, USH1C, RDX, DCD C2, LOXHD1, TPRN, SERPINB6, SiX1, CDH23, WHRN, OtoGL, WFS1, MYH9, REST, MCM2, DMXL2, TMIE, PCDH15, GRXC R1, TRIOBP, ILDR1, CIB2, GPSM2, ELMOD3, CABP2, TMEM132E, GRXCR2, EPS8, CLIC5, TRIOBP, ILDR1, SLC26A5.

In one or more embodiments, the disease is hereditary deafness caused by a gene defect in the cochlear hair cells. Preferably, the disease is sensorineural hearing loss. In one or more embodiments, the sensorineural hearing loss comprises congenital sensorineural deafness. In one or more embodiments, the congenital sensorineural deafness is congenital hereditary sensorineural deafness.

In one or more embodiments, the subject is a mammal, such as a mouse, rat, rabbit, dog, pig, monkey or human, preferably a pig or human.

### Beneficial Effects of the Present Invention:

The promoter provided by the invention can be specifically expressed in hair cells, and can perform gene therapy for hearing loss caused by deafness gene mutation.

### DESCRIPTION OF FIGURES

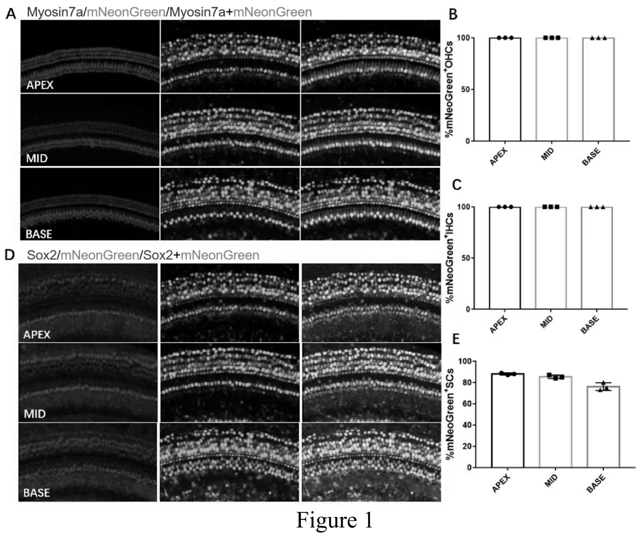

Figure 1 shows the expression of reporter genes driven by the broad-spectrum promoter CAG in cochlear cells.
   A: AAV-ie-CAG-mNeonGreen-Bpnls virus (Bpnls is used to express mNeonGreen in the nucleus) was injected into the round window of P2 wild-type mice, and cochlear samples were collected two weeks later for immunofluorescence staining of cochlear basilar membrane. The experiment shows the expression of mNeonGreen reporter gene driven by CAG promoter in cochlear hair cells. The mNeonGreen reporter gene is co-labeled with the hair cell marker Myosin7a, indicating that CAG promoter drives the reporter gene to be widely expressed in cochlear hair cells.
   B: A statistical chart showing the infection status of the mNeonGreen reporter gene in cochlear outer hair cells, indicating that the infection efficiency of the CAG promoter-driven reporter gene in cochlear outer hair cells (OHC) is close to 100%.
   C: A statistical chart showing the infection status of the mNeonGreen reporter gene in cochlear inner hair cells, indicating that the infection efficiency of the CAG promoter-driven reporter gene in cochlear inner hair cells (IHC) is close to 100%.
   D: AAV-ie-CAG-mNeonGreen-Bpnls virus was injected into the round window of P2 wild-type mice, and cochlear samples were collected two weeks later for immunofluorescence staining of cochlear basement membrane. The experiment showed the expression of mNeonGreen reporter gene driven by CAG promoter in cochlear supporting cells. The mNeonGreen reporter gene was co-labeled with supporting cell marker Sox2, indicating that CAG promoter drives the reporter gene to be widely expressed in cochlear supporting cells (SC).
   E: A statistical diagram of the infection status of the mNeonGreen reporter gene in cochlear supporting cells, indicating that the infection efficiency of the CAG promoter-driven reporter gene in cochlear supporting cells (SC) can reach more than 75%.

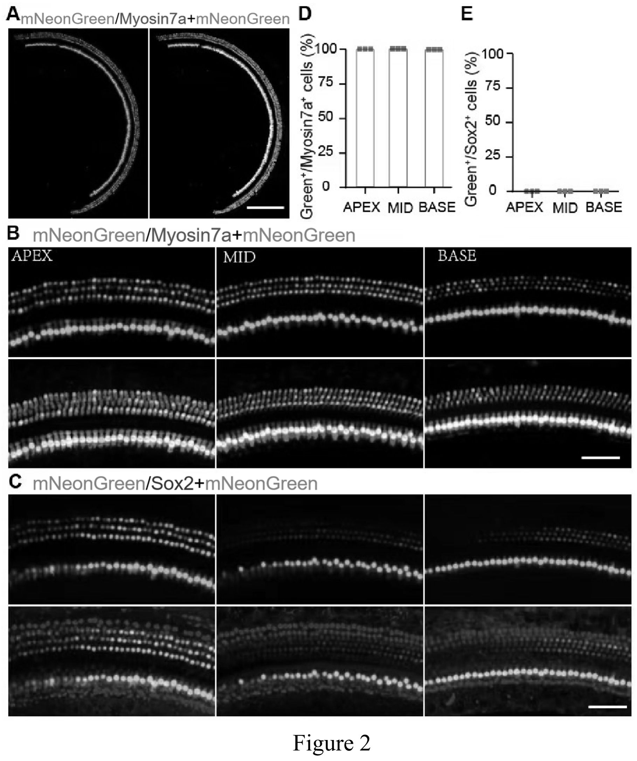

Figure 2 shows immunofluorescence staining of the cochlea taken from newborn mice on the 14th day after the virus AAV-ie-Otov-HC-mNeonGreen was injected into the cochlea through the round window at P3. mNeonGreen was only expressed in the inner hair cells (IHC) and outer hair cells (OHC) of the cochlea, but not in supporting cells (SC) and other cells.
   A: AAV-ie-Otov-HC-mNeonGreen-BpnIs virus was injected into the round window of P3 wild-type mice, and cochlear samples were collected two weeks later for immunofluorescence staining of cochlear basement membrane. The experiment shows the expression of mNeonGreen reporter gene driven by Otov-HC specific promoter in cochlear hair cells. The mNeonGreen reporter gene is co-labeled with the hair cell marker Myosin7a, indicating that Otov-HC specific promoter drives the specific expression of the reporter gene only in cochlear hair cells.
   B: AAV-ie-Otov-HC-mNeonGreen-BpnIs virus was injected into the round window of P3 wild-type mice, and cochlear samples were collected two weeks later for immunofluorescence staining of cochlear basement membrane. The experiment shows the expression of mNeonGreen reporter gene driven by Otov-HC specific promoter in cochlear hair cells. The mNeonGreen reporter gene is co-labeled with the hair cell marker Myosin7a, indicating that Otov-HC specific promoter drives the specific expression of the reporter gene only in cochlear hair cells.
   C: AAV-ie-Otov-HC-mNeonGreen-BpnIs virus was injected into the round window of P3 wild-type mice, and cochlear samples were collected two weeks later for immunofluorescence staining of cochlear basement membrane. The experiment shows the expression of mNeonGreen reporter gene driven by Otov-HC specific promoter in cochlear supporting cells. The mNeonGreen reporter gene was not co-labeled with supporting cell marker Sox2, indicating that Otov-HC specific promoter drove the reporter gene not to be expressed in cochlear supporting cells.
   D: A statistical chart showing the infection of the mNeonGreen reporter gene driven by the Otov-HC specific promoter in cochlear hair cells, indicating that the infection efficiency of the reporter gene driven by the Otov-HC specific promoter in cochlear hair cells can reach 100%.
   E: A statistical graph of the infection of mNeonGreen reporter gene driven by Otov-HC specific promoter in cochlear supporting cells, indicating that the infection efficiency of Otov-HC specific promoter driven reporter gene in cochlear supporting cells is almost zero.

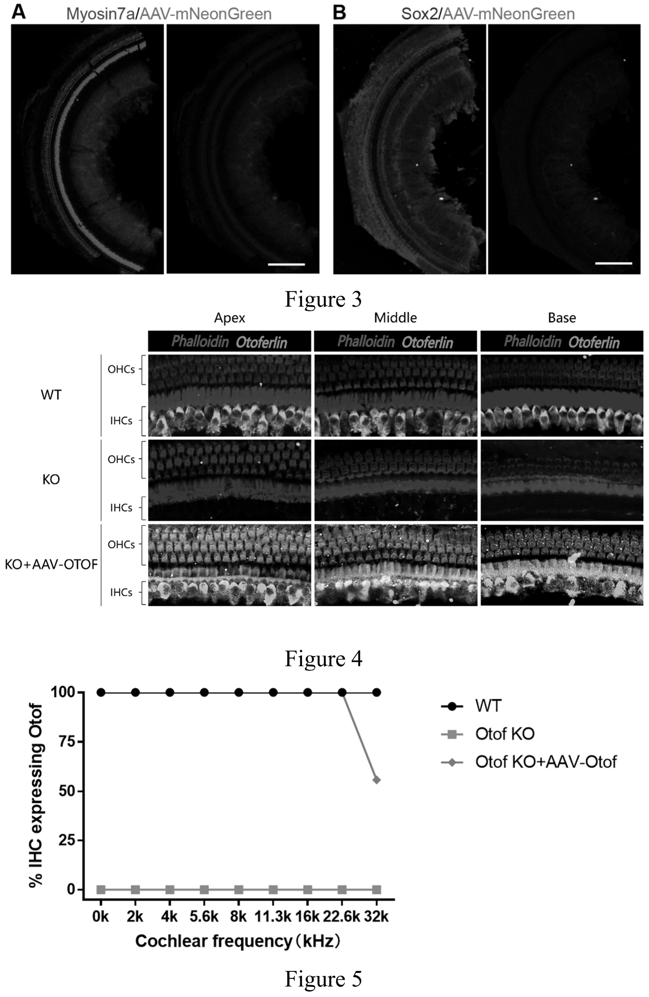

Figure 3 shows that the SHH sequence alone drives gene expression in cochlear cells.
   A: Shows the situation where the SHH sequence alone drives gene expression in cochlear hair cells.
   B: Shows the situation where the SHH sequence alone drives gene expression in cochlear supporting cells.
Figure 4 shows the expression of the exogenous Otof gene driven by the Otov-HC specific promoter in the cochlear cells of OtofKO mice, indicating that the Otov-HC specific promoter drives the Otof gene to be specifically expressed only in the cochlear hair cells.
Figure 5 shows a statistical diagram of the expression efficiency of the exogenous Otof g ene driven by the Otov-HC specific promoter. The Otov-HC specific promoter drives the Otof ge ne to express at low and medium frequencies with a recombination efficiency of 100%, and at high frequencies with a recombination expression efficiency of more than 55%.

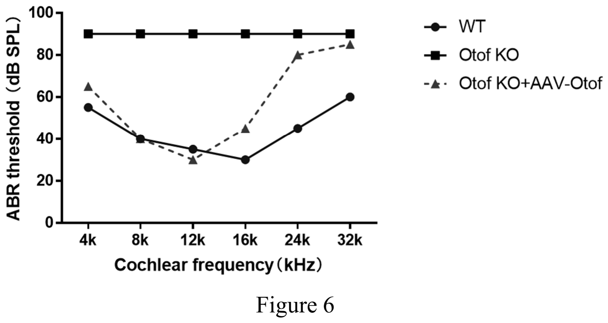

Figure 6 shows the ABR results. It shows that after knocking out the Otof gene, the hear ing threshold of the mice increased significantly at low, medium and high frequencies, indicating complete deafness. After receiving dual-vector Otof gene therapy, the ABR threshold decrease d, and each hearing frequency band was significantly restored, with the hearing recovery effect similar to that of WT mice in the same litter.

### DETAILED DESCRIPTION

The practice of the present description will employ, unless otherwise indicated, conventional techniques of molecular biology (including recombinant techniques), microbiology, cell biology, biochemistry and immunology, which are within the skill of the art. Such techniques are explained fully in the literature, such as, Molecular Cloning: A Laboratory Manual, second edition (Sambrook et al., 1989); Oligonucleotide Synthesis (MJ. Gait, ed., 1984); Animal Cell Culture (RJ. Freshney, ed., 1987); Methods in Enzymology (Academic Press, Inc.); Current Protocols in Molecular Biology (F.M. Ausubel et al., eds 1987, and periodic updates); PCR: The Polymerase Chain Reaction, (Mullis et al., ed., 1994); A Practical Guide to Molecular Cloning (Perbal Bernard V., 1988); Phage Display: A Laboratory Manual (Barbas et al., 2001).

Compared with the prior art, the present invention provides a treatment method that specifically targets cochlear hair cells: a promoter that can specifically target cochlear hair cells is used to allow a viral vector containing the promoter to deliver a specific transgene (such as ATOH1, VGLUT3, TMC1/2, KCNQ1, MLRB3, LHFPL5, OTOF, STRC, MYO7A, CHD7, TMPRSS3, POU3F4, ESPN, SYNE4, DIAPH1, KCNQ4, CEACAM16, MYO6, P2RX2, SMAC, TBC1D24, CD164, OSBPL2, HOMER2, MYO3A, PDE1C, SCD5, US H1C, RDX, DCDC2, LOXHD1, TPRN, SERPINB6, SiX1, CDH23, WHRN, OtoGL, WFS1, MYH9, REST, MCM2, DMXL2, TMIE, PCDH15, GRXCR1, TRIOBP, ILDR1, CIB2, GPSM2, ELMOD3, CABP2, TMEM132E, GRXCR2, EPS8, CLIC5, TRIOBP, ILDR1, SLC26A5, etc.) and gene therapy tools target cochlear hair cells to promote the expression of transgenes in hair cells, thereby compensating for the lack of related proteins and achieving the purpose of treating hereditary deafness.

### Specific promoter

The inventors have discovered a promoter that can specifically activate gene expression in cochlear hair cells, which has a sequence shown in SEQ ID NO: 1 or a homologous sequence having at least 90% identity thereto. Based on this, compared with the prior art, the present invention provides a cochlear hair cell-specific promoter and a treatment method that specifically targets cochlear hair cells: by using a promoter that can specifically target cochlear hair cells, a viral vector containing the promoter targets a specific gene to cochlear hair cells, promotes the expression of a transgene in cochlear hair cells, and thus performs gene therapy for hearing loss caused by deafness gene mutations.

A promoter is a DNA sequence that RNA polymerase recognizes, binds to, and initiates transcription, and it is located upstream of the start codon of a structural gene (usually within about 100 to 1000 bp) that controls the transcription of the structural gene. It contains conserved sequences required for specific binding of RNA polymerase and transcription initiation, and the promoter itself is not transcribed. Its general structure includes core promoter elements and upstream regulatory elements. The core promoter element refers to the minimum DNA sequence required for RNA polymerase to initiate transcription. The core promoter elements of eukaryotic organisms include the transcription start point and the TATA box located 25 to 30 bp upstream of it, which mainly serves as a site for RNA polymerase to bind and initiate transcription. When the core promoter element acts alone, it can determine the transcription start site and produce a basal level of transcription. The upstream regulatory element can change the efficiency of transcription by binding to the corresponding trans-acting factor.

In some embodiments, the cochlear hair cell-specific promoter of the present invention comprises: (1) an enhancer sequence shown in SEQ ID NO: 2 or a sequence having at least 90% sequence identity thereto and retaining the function of SEQ ID NO: 2, and (2) a core promoter element as shown in SEQ ID NO: 3 or a homologous sequence having at least 90% sequence identity thereto. In one or more embodiments, the enhancer sequence described in (1) is located upstream or downstream of the core promoter element described in (2).

In the present invention, the specific promoter can act as a promoter to specifically guide gene expression in cochlear hair cells, and the core promoter element described in (2) is a core promoter element suitable for promoting gene expression in mammalian cells. The sequences of these core promoter elements can be easily obtained by those skilled in the art. According to some embodiments, the homologous sequence described in (2) is the sequence of the core promoter element of the SHH gene of mammals, preferably the SHH gene of primates, more preferably the SHH gene of hominids.

In one or more embodiments, the promoter comprises the sequence shown in SEQ ID NO: 1, or a sequence having at least 90% sequence identity thereto.

In one or more embodiments, the sequence shown in SEQ ID NO: 1 consists of two sequences, one of which is derived from the human genome sequence shown in SEQ ID NO: 2. The other is derived from the core promoter element of the human SHH gene, and its sequence is shown in SEQ ID NO: 3.

The present invention also relates to homologous sequences having at least 50%, at least 70%, at least 80%, at least 90%, at least 95%, at least 98%, at least 99% identity with the above-mentioned nucleic acid sequences (e.g., sequences of the components in the specific promoter). Herein, "homologous sequence" refers to a sequence from a species of the same order, family, genus, or species as the species of the source sequence and having the sequence identity.

The present invention also relates to a polynucleotide that hybridize with the of the above-mentioned specific promoter and has at least 50%, preferably at least 70%, more preferably at least 80% identity between the two sequences. The description particularly relates to polynucleotides that can hybridize with the polynucleotides of the description under strict conditions. In the present description, "strict conditions" refer to: (1) hybridization and elution at lower ionic strength and higher temperature, such as 0.2 × SSC, 0.1%SDS, 60 °C; or (2) addition of denaturants during hybridization, such as 50% (v/v) formamide, 0.1% calf serum/0.1% Ficoll, 42 °C, etc; or (3) hybridization that occurs only when the identity between two sequences is at least more than 90%, or preferably, more than 95%.

### Nucleic acid construct

The present invention also provides nucleic acid constructs comprising the above-mentioned specific promoters for genetic functions such as cloning or gene expression. Nucleic acid constructs can be expression cassettes or vectors. Therefore, in addition to the specific promoter, nucleic acid constructs can also include other elements required for cloning or expression, such as multiple cloning sites and/or terminators (such as polyA signal sequences). For example, nucleic acid constructs can be RNA constructs or DNA vectors that can be transfected cells and expressed. Herein, "nucleic acid molecules", "polynucleotides", and "nucleic acids" are used interchangeably.

In order to express a polypeptide or protein, the nucleic acid construct further comprises a gene of interest, which is operably linked to the specific promoter. Herein, a gene may be a coding sequence that can encode a polypeptide or protein. In one or more embodiments of the present invention, gene is selected from: (1) ATOH1, VGLUT3, TMC1/2, KCNQ1, MLRB3, LHFPL5, OTOF, STRC, MYO7A, CHD7, TMPRSS3, POU3F4, ESPN, SYNE4, DIAPH1, KCNQ4, CEACAM16, MYO6, P2RX2, SMAC, TBC1D24, CD164, OSBPL2, HOMER2, MYO3A, PDE1C, SCD5, USH1C, RDX, DCD C2, LOXHD1, TPRN, SERPINB6, SiX1, CDH23, WHRN, OtoGL, WFS1, MYH9, REST, MCM2, DMXL2, TMIE, PCDH15, GRXC R1, TRIOBP, ILDR1, CIB2, GPSM2, ELMOD3, CABP2, TMEM132E, GRXCR2, EPS8, CLIC5, TRIOBP, ILDR1, SLC26A5, etc. The gene can also be various tool genes for operating the genes of cochlear hair cells or expressing in cochlear hair cells, such as CRSPR-Cas system, Talen, ZFN and other gene editing tools, and tool genes involved in technologies such as shRNA, DNA/RNA base editing systems, and prime editing, for example, gRNA and Cas enzyme protein expression genes. These techniques and tool genes are common knowledge for those skilled in the art. The polynucleotides described herein are operably connected to therapeutic transgenes to provide a theoretical basis for clinical targeted treatment of hereditary hearing loss.

In the case where a gene or its sequence is known, the coding nucleic acid of each gene can be prepared by a conventional method in the art, and a corresponding vector can be constructed. Recombinant vectors can be constructed using methods well known to those skilled in the art, see, for example, Sambrook et al. (2001, Molecular Cloning: A Laboratory Manual, Cold Spring Harbor Laboratory), Ausubel et al. (1989, Short Protocols in Molecular Biology, Wiley) or other standard textbooks described in the technology. Alternatively, the nucleic acid construct can be reconstructed into a liposome for delivery to a target cell. Containing the nucleic acid construct of the present invention can be transferred to a host cell by a well-known method, which varies according to the type of the cell host. For example, calcium chloride transfection is commonly used for prokaryotic cells, and calcium phosphate treatment or electroporation can be used for other cell hosts, see Sambrook et al. (see above).

The vector described herein contains a nucleic acid molecule or expression cassette described herein. The vector can be a plasmid, a cosmid, a virus, or a viral vector. The vector includes a cloning vector, an integration vector, or an expression vector. The expression vector generally contains sequences for plasmid maintenance and for cloning and expressing exogenous nucleotide sequences. The sequences (collectively referred to as "flanking sequence" in some embodiments) generally comprises one or more of the following nucleotide sequences: promoter (e.g., a specific promoter described herein), one or more enhancer sequences, replication origin, transcription termination sequence, complete intronic sequence comprising donor and receptor splice sites, sequence encoding leader sequence for polypeptide secretion, ribosome binding site, polyadenylation sequence, a multiple cloning site for inserting a nucleic acid encoding a protein to be expressed and an optional marker element. Typically, the promoter is located at the 5' end of the multiple cloning site or the polypeptide coding sequence. In one or more embodiments, the expression vector uses an AAV series vector as a backbone.

The vector may optionally contain a "tag" coding sequence, i.e., an oligonucleotide molecule located at the 5' or 3' end of the polypeptide coding sequence; the oligonucleotide sequence encodes polyhistidine (such as 6His) or another "tag", such as FLAG, HA or myc. This tag is typically fused to the polypeptide when it is expressed, and can serve as a means for affinity purification or detection of the protein from host cells. Affinity purification can be accomplished, for example, by column chromatography using an antibody against this tag as an affinity matrix. The tag can optionally be subsequently removed from the purified protein by various means, such as using certain peptidases for cleavage.

Flanking sequences can be homologous (i.e., from the same species and / or strain as the host cell), heterologous (i.e., from a species other than the host cell species or strain), heterozygous (i.e., from a combination of flanking sequences from the above sources), synthetic or natural. Flanking sequences can be homologous (i.e., from the same species and / or strain as the host cell), heterologous (i.e., from a species other than the host cell species or strain), heterozygous (i.e., from a combination of flanking sequences from the above sources), synthetic or natural. Similarly, the source of the flanking sequences can be any prokaryotic or eukaryotic organism, any vertebrate or invertebrate organisms or any plant, provided that the flanking sequences are functional and activatable by host cell machinery.

The selectable marker gene encodes a protein necessary for the survival and growth of host cells grown in selective media. Typical selectable marker genes encode (a) confer resistance to antibiotics or other toxins (e.g., ampicillin, tetracycline or kanamycin for prokaryotic host cells); (b) complement the auxotrophy of the cells; or (c) proteins that provide important nutrients not available from complexes or defined media. Specific selectable markers are the kanamycin resistance gene, the ampicillin resistance gene and the tetracycline resistance gene. Advantageously, the neomycin resistance gene can also be used for selection in prokaryotic and eukaryotic host cells.

### AAV vectors and AAV particles

The term "adeno-associated virus" or "AAV" as used herein includes members of the class of viruses associated with the name, which belongs to the genus Dependoparvovirus, family Parvoviridae. Adeno-associated virus is a single-stranded DNA virus that grows exclusively in cells, where certain functions are provided by a co-infecting helper virus. General information and reviews of AAV can be found, for example, in Carter, 1989, Handbook of Parvoviruses, Vol. 1, pp. 169-228. Various serotypes of the virus are known to be suitable for gene delivery, and the various serotypes are closely related in structure and function. All AAV serotypes apparently exhibit very similar replication characteristics mediated by homologous rep genes; and all carry three related capsid proteins. At least 13 sequentially numbered AAV serotypes are known in the art. Non-limiting exemplary serotypes that can be used in the methods disclosed herein include any of the 13 serotypes, such as AAV2, AAV8, AAV9. AAV particles comprise, consist essentially of, or consist of the following three major viral proteins: VP1, VP2, and VP3. In one or more embodiments, AAV refers to serotype AAV1, AAV2, AAV4, AAV5, AAV6, AAV7, AAV8, AAV9, AAV10, AAV11, AAV12, AAV13, AAVPHP.B, or AAVrh74.

In some embodiments, the nucleic acid construct of the present invention is a pAAV vector that expresses a gene of interest through a recombinant AAV vector system. "pAAV vector" as used herein refers to a vector that comprises, essentially consists of, or consists of a specific promoter and one or more AAV inverted terminal repeats (ITRs) as described herein. When present in a host cell that provides functionality of the rep and cap gene products, the pAAV vector can be replicated and packaged into infectious viral particles; for example, by transfecting a host cell. In one or more embodiments, the pAAV vector contains a specific promoter described herein, at least one nucleic acid encoding at least one protein or RNA, and/or an enhancer and/or terminator packaged into the flanking ITRs of infectious AAV particles. The plasmid containing the pAAV vector may also contain elements for preparation purposes, such as antibiotic resistance genes, etc.

The present invention also provides an AAV vector system, comprising the pAAV vector and other nucleic acid constructs encoding genes required by the AAV virus, wherein the pAAV vector comprises a specific promoter as described in any embodiment of the present invention. In one or more embodiments, the pAAV vector further comprises a gene of interest, which is operably linked to the specific promoter. In one or more embodiments, the AAV vector system comprises an Anc80L65 plasmid, a helper plasmid, and the pAAV vector.

As used herein, the term "viral capsid" or "capsid" refers to the protein shell or capsid of the virus particle. The function of the capsid is to encapsidate, protect, transport the viral genome, and release it into the host cell. The capsid is usually composed of oligomeric structural subunits of proteins ("capsid proteins") As used herein, the term "encapsid" refers to being contained within the viral capsid. The viral capsid of AAV is composed of a mixture of three viral capsid proteins: VP1, VP2 and VP3.

"AAV virus particle" or "AAV particle" refers to a virus particle composed of at least one AAV capsid protein and a polynucleotide pAAV vector encapsidated therein. Therefore, the production of AAV particles necessarily includes the production of pAAV vectors, because such vectors are contained in AAV particles. The present invention provides an AAV virus particle comprising a specific promoter as described herein, and optionally further comprising a gene of interest operably linked to the specific promoter.

As used herein, the term "helper" refers to a virus or plasmid refers to a virus or plasmid that is used to provide additional components necessary for replication and packaging of any of the pAAV vectors described herein. The components encoded by the helper virus may include any genes required for virion assembly, encapsidation, genome replication and/or packaging. For example, a helper virus or plasmid may encode an enzyme necessary for viral genome replication. Non-limiting examples of helper viruses and plasmids suitable for use with pAAV constructs include pHelper (plasmid), adenovirus (virus), or herpes virus (virus).

Methods for producing and using AAV vectors are known in the art. Methods for producing AAV particles and heterologous nucleic acids are also known in the art and are commercially available (see, e.g., US 2007/0015238 and US2012/0322861, which are incorporated herein by reference in their entirety). For example, a plasmid comprising a heterologous nucleic acid sequence can be combined with one or more helper plasmids (e.g., comprising rep genes (e.g., encoding Rep78, Rep68, Rep52, and Rep40) and cap genes (encoding VP1, VP2, and/or VP3) and transfected or permanently integrated into a production cell line so that the AAV particles can be packaged and subsequently purified.

In some embodiments, one or more helper plasmids include a first helper plasmid comprising a rep gene and a cap gene and a second helper plasmid comprising an E1a gene, an E1b gene, an E4 gene, an E2a gene, and a VA gene. In some embodiments, the rep gene is a rep gene derived from AAV2, and the cap gene is derived from AAV44.9. Helper plasmids and methods for preparing such plasmids are known in the art and are commercially available.

### Cell

The present invention also includes host cells that contain or have integrated into their genome a specific promoter or nucleic acid construct as described herein. Host cells can be used as recipients of vectors. Host cells can be "transfected" or "transformed," which refers to the process of transfection or transduction of exogenous nucleic acids into host cells. Transformed cells include primary subject cells and their progeny. The terms "engineered" and "recombinant" cells or host cells used herein often refer to cells into which exogenous nucleic acid sequences, such as vectors, have been introduced. Therefore, recombinant cells can be distinguished from naturally occurring cells that do not contain the introduced recombinant nucleic acids.

Suitable host cells are as described above, including but not limited to Chinese hamster ovary (CHO) cells, HeLa cells, baby hamster kidney (BHK) cells, monkey kidney cells (COS), human hepatocellular carcinoma cells (eg, HepG2), cochlear hair cells, and the like.

As used herein, packaging cells (or helper cells) are cells for producing viral vectors. Production of recombinant AAV viral vectors requires trans-provided Rep and Cap proteins and gene sequences from adenovirus-assisted AAV replication. In some aspects, packaging/helper cells contain plasmids that are stably integrated into the cell genome. In other aspects, packaging cells can be transiently transfected. Typically, packaging cells are eukaryotic cells, such as mammalian cells or insect cells. In one or more embodiments, the cell for producing viral vectors is a HEK293 cell, including HEK293T, HEK293H, HEK293F, HEK293S, HEK293T/17, HEK293T/17SF, HEK293FT, HEK293SG, HEK293E, HEK293-6E, HEK293FTM, HEK293SGGD cells.

The nucleic acid construct/recombinant expression vector of the present invention can be transferred into the cell of interest. The method of transfer is a conventional method in the art, including but not limited to: viral transduction, microinjection, particle bombardment, gene gun transformation and electrotransformation. In some embodiments, electrotransformation is used to transfer the nucleic acid construct or recombinant expression vector. When the host cell is cultured under appropriate conditions, it synthesizes the fusion protein, which can then be collected from the culture medium (if the host cell secretes it into the culture medium) or directly collected from the host cell that produces it (if it is not secreted).

### Methods for preparing AAV vectors and viruses

A variety of methods can be used to prepare AAV viral vectors. In one or more embodiments, packaging is achieved by using a helper virus or a helper plasmid and a cell line. The helper virus or helper plasmid contains elements and sequences that promote the production of viral vectors. In some embodiments, the helper plasmid is stably integrated into the genome of the packaging cell line so that the packaging cell line does not need to be additionally transfected with the helper plasmid.

In one or more embodiments, the cell is a packaging cell line or a helper cell line. In one or more embodiments, the helper cell line is a eukaryotic cell; for example, a HEK 293 cell or a 293T cell. In one or more embodiments, the helper cell is a yeast cell or an insect cell.

The helper plasmid can comprise, for example, at least one viral helper DNA sequence derived from a replication-incompetent viral genome encoding trans-acting full virion proteins required for packaging of replication-incompetent AAV, and virion proteins for producing virions capable of packaging replication-incompetent AAV at high titers without producing replication-incompetent AAV.

Helper plasmids for packaging AAV are known in the art, see, for example, U.S. Patent Publication No. 2004/0235174A1, which is incorporated herein by reference. As described herein, as a non-limiting example, the AAV helper plasmid may contain the Ad5 genes E2A, E4 and VA controlled by respective native promoters or heterologous promoters as hepler viral DNA sequences. The AAV helper plasmid may additionally contain an expression cassette for expressing a marker protein such as a fluorescent protein to allow easy detection of transfection of desired target cells.

Provided herein is a method for producing AAV particles, comprising transfecting a packaging cell line with any of the AAV helper plasmids disclosed herein; and any of the AAV vectors disclosed herein. In some embodiments, the AAV helper plasmid and the AAV vector are co-transfected into the packaging cell line. In some embodiments, the cell line is a mammalian cell line, such as a human embryonic kidney (HEK) 293 cell line. Provided herein is a cell comprising any of the AAV vectors and/or AAV particles disclosed herein.

### Pharmaceutical composition

The present invention also provides a pharmaceutical composition comprising a pharmaceutically acceptable excipient and one or more selected from the group consisting of: a specific promoter, a nucleic acid construct, an AAV vector system, an AAV virus particle, and a host cell as described herein. In one or more embodiments, the pharmaceutical composition comprises a pAAV vector and a pharmaceutically acceptable excipient, wherein the pAAV vector comprises a specific promoter described herein operably linked to a gene of interest. In one or more embodiments, the pharmaceutical composition comprises an AAV virus particle and a pharmaceutically acceptable excipient, wherein the AAV virus particle comprises a polynucleotide comprising a specific promoter as described herein operably linked to a gene of interest.

As used herein, the term "pharmaceutically acceptable excipient" encompasses any standard pharmaceutical carrier, such as phosphate buffered saline solution, water and emulsions, such as oil/water or water/oil emulsions, and various types of wetting agents. The composition may also include stabilizers and preservatives. For examples of carriers, stabilizers and adjuvants, see Martin (1975) Remington's Pharm. Sci. 15th Edition (Mack Publ. Co., Easton).

As described herein, pharmaceutical compositions can be formulated by any method known or developed in the field of pharmacology, including but not limited to contacting an active ingredient (e.g., a viral particle or a recombinant vector) with an excipient or other auxiliary ingredient, dividing or packaging the product into dosage units. The viral particles herein can be formulated to have desired characteristics, such as increased stability, increased cell transfection, sustained or delayed release, biodistribution or tropism, regulation of the encoded protein in vivo or enhanced translation, and release profiles of the encoded protein in vivo.

Thus, the pharmaceutical composition can further comprise saline, lipidoids, liposomes, lipid nanoparticles, polymers, lipid complexes, core-shell nanoparticles, peptides, proteins, cells transfected with viral vectors (e.g., for transplantation into a subject), nanoparticle mimics, or a combination thereof. In some embodiments, the pharmaceutical composition is formulated as nanoparticles. In some embodiments, the nanoparticles are self-assembled nucleic acid nanoparticles.

According to the pharmaceutical composition herein, it can be prepared, packaged and/or sold in bulk with a single unit dose and/or with multiple single unit doses. The amount of the active ingredient is generally equal to the dosage of the active ingredient to be administered to the subject and/or the appropriate ratio of such dosage, such as half or one-third of such dosage. The preparation of the present invention may include one or more excipients, and the amount of each excipient together increases the stability of the viral vector, increases cell transfection or viral vector transduction, increases the expression of the protein encoded by the viral vector, and/or changes the release profile of the protein encoded by the viral vector. In some embodiments, the pharmaceutical composition includes an excipient. Non-limiting examples of excipients include solvents, dispersion media, diluents or other liquid vehicles, dispersion or suspension aids, surfactants, isotonic agents, thickeners or emulsifiers, preservatives or combinations thereof.

In some embodiments, the pharmaceutical composition comprises a cryoprotectant. The term "cryoprotectant" refers to an agent that can reduce or eliminate damage to a substance during freezing. Non-limiting examples of cryoprotectants include sucrose, trehalose, lactose, glycerol, dextrose, raffinose and/or mannitol.

### Uses and Methods

The present invention provides the use of the specific promoter in the preparation of a drug for treating a disease caused by a gene defect in cochlear hair cells, wherein the drug is used for expressing the gene in the cochlear hair cells.

The present invention provides a method for introducing a target gene into a subject's cell, comprising contacting the cell with an effective amount of any AAV virus particle described herein, wherein the particle comprises a polynucleotide comprising a specific promoter described herein operably linked to the gene of interest.

The present invention provides a method for expressing a gene of interest in cochlear hair cells, comprising the step of introducing a nucleic acid construct comprising the gene into cochlear hair cells, wherein the gene is operably linked to a specific promoter described herein. Typically, the promoter is in the same expression frame as the gene and is located upstream of the gene. Examples of genes of interest are described elsewhere herein.

The specific promoter described herein is operably connected to therapeutic genes to provide a theoretical basis for clinical targeted treatment of hereditary hearing loss. The present invention provides a method for treating a disease caused by a gene defect in cochlear hair cells, comprising the step of expressing the gene in the cochlear hair cells, wherein the gene is operably linked to the cochlear hair cell-specific promoter described herein. The method comprises the step of introducing a nucleic acid construct or vector comprising the coding sequence of the gene into the cochlear hair cells, wherein the coding sequence of the gene is operably linked to the specific promoter. Exemplarily, the gene is selected from: a deafness gene, a tool gene for manipulating genes of cochlear hair cells or expressed in cochlear hair cells. In one or more embodiments, the deafness gene is a deafness gene expressed in cochlear hair cells. In one or more embodiments, the deafness gene is a causative gene for non-syndromic deafness. In one or more embodiments, the deafness gene includes one or more selected from the group consisting of: ATOH1, VGLUT3, TMC1/2, KCNQ1, MLRB3, LHFPL5, OTOF, STRC, MYO7A, CHD7, TMPRSS3, POU3F4, ESPN, SYNE4, DIAPH1, KCNQ4, CEACAM16, MYO6, P2RX2, SMAC, TBC1D24, CD164, OSBPL2, HOMER2, MYO3A, PDE1C, SCD5, USH1C, RDX, DCD C2, LOXHD1, TPRN, SERPINB6, SiX1, CDH23, WHRN, OtoGL, WFS1, MYH9, REST, MCM2, DMXL2, TMIE, PCDH15, GRXC R1, TRIOBP, ILDR1, CIB2, GPSM2, ELMOD3, CABP2, TMEM132E, GRXCR2, EPS8, CLIC5, TRIOBP, ILDR1, SLC26A5. The disease is hereditary deafness caused by a gene defect in cochlear hair cells. Preferably, the disease is sensorineural hearing loss. Herein, "hearing loss" and "deafness" are used interchangeably. Congenital deafness in sensorineural deafness includes non-hereditary and hereditary. Among them, 70% of hereditary patients have no other symptoms except deafness, which is called non-syndromic deafness. Therefore, sensorineural hearing loss includes congenital hearing loss and non-congenital hearing loss.

The term "disease and/or condition" refers to the physical state of a subject associated with a disease and/or condition described herein. "Treatment" as used herein includes any beneficial or desired effect on the symptoms or pathology of a disease or pathological condition, and may include even a small reduction in one or more measurable markers of the disease or condition (e.g., a hereditary deafness disease) being treated. Treatment may optionally include a reduction or alleviation of the symptoms of a disease or condition, or a delay in the progression of the disease or condition. "Treatment" does not necessarily mean complete eradication or cure of the disease or condition or its associated symptoms. As used herein, "treatment" of a disease in a subject refers to (1) prevention of symptoms or disease in a subject predisposed to or not yet manifesting symptoms of the disease; (2) suppression of the disease or inhibition of its progression; or (3) amelioration or induction of regression of the disease or its symptoms. As understood in the art, "treatment" is a method for obtaining beneficial or desired results, including clinical results. Beneficial or desired results may include one or more, but are not limited to, alleviation or amelioration of one or more symptoms, whether detectable or undetectable; reduction in the extent of a condition (including a disease); stabilization (i.e., non-worsening) of a condition (including disease); delay or slowing of the progression of a condition (including disease); improvement or palliation of a condition (including disease) and its status; and remission (whether partial or total).

The term "subject" or "patient" may refer to a cell or animal, such as a mammal or a human, that receives a pharmaceutical composition of the present invention to treat, prevent, improve and/or alleviate a disease or condition of the present invention. The subject is not limited to a particular species and include non-human animals undergoing diagnosis or treatment, as well as those subjected to infection or modeling., including but not limited to dog, monkey, cow, horse, ape, mouse, rat, canine or rabbit species, and other livestock, sports animals or pets. In some embodiments, the subject is a human.

The term "effective amount" as used herein is intended to mean an amount sufficient to achieve the desired effect. In the case of therapeutic or preventive applications, the effective amount will depend on the type and severity of the disorder in question and the characteristics of the individual subject, such as general health, age, sex, weight, and tolerance to the pharmaceutical composition. In the context of gene therapy, in some embodiments, the effective amount is an amount sufficient to restore some or all of the function of a defective gene in a subject. In some other embodiments, the effective amount of AAV viral particles is an amount sufficient to cause the gene to be expressed in a subject. In some embodiments, the effective amount is an amount sufficient to enhance galactose metabolism in a subject in need. A skilled technician will be able to determine the appropriate amount based on these and other factors.

In some embodiments, the effective amount will depend on the scale and nature of the application in question. It also depends on the nature and sensitivity of the target subject as well as the methodology employed. Those skilled in the art will be able to determine the effective amount based on these and other considerations. According to an embodiment, the effective amount may include, consist essentially of, or consist of one or more administrations of the composition.

As used herein, the term "administering" refers to the process of delivering a substance to a subject, such as an animal or a human. Administration can be performed in one dose, continuously or intermittently throughout the course of treatment. Methods for determining the most effective mode of administration and dosage are known to those skilled in the art and will vary with the composition used for treatment, the purpose of treatment, and the age, health or sex of the subject being treated. Single or multiple administrations can be performed, with dosage levels and patterns selected by the treating physician, or in the case of pets and other animals, by the treating veterinarian.

### Dosage and Administration

Methods for determining the most effective mode of administration and dosage are known to those skilled in the art and will vary with the composition used for treatment, the purpose of the treatment, and the subject being treated. Single or multiple administrations may be performed, with the dosage level and pattern being selected by the treating physician. The dosage may be affected by the route of administration. Suitable dosage formulations and methods of administering the agents are known in the art. Non-limiting examples of such suitable dosages may be as low as 10E9 vector genomes to as high as 10E17 vector genomes per administration.

In embodiments of the methods described herein, the number of viral particles (e.g., AAV) administered to a subject ranges from about 10E9 to about 10E17. In certain embodiments, about 10E10 to about 10E12, about 10E11 to about 10E13, about 10E11 to about 10E12, about 10E11 to about 10E14, about 5×10E11 to about 5×10E12, or about 10E12 to about 10E13 viral particles are administered to a subject.

In some embodiments, the AAV particles repair a genetic defect in a subject. In some embodiments, the ratio of repaired target polynucleotides (e.g., Myo7a, STRC, OTOF) or polypeptides to unrepaired target polynucleotides or polypeptides in successfully treated cells, tissues, organs, or subjects is at least about 1.5:1, about 2: 1, about 3: 1, about 4: 1, about 5:1, about 6:1, about 7:1, about 8:1, about 9:1, about 10:1, about 20:1, about 50:1, about 100:1, about 1000:1, about 10,000:1, about 100,000:1, or about 1,000,000:1. The amount or ratio of the repaired target polynucleotide or polypeptide can be determined by any method known in the art, including but not limited to Western blotting, Northern blotting, Southern blotting, PCR, sequencing, mass spectrometry, flow cytometry, immunohistochemistry, immunofluorescence, fluorescence in situ hybridization, sequencing, immunoblotting, and ELISA.

In some embodiments, the viral particles are introduced into the subject via intravenous, intrathecal, intracerebral, intraventricular, intranasal, intratracheal, intraotic, intraocular or periocular, oral, rectal, transmucosal, inhalational, transdermal, parenteral, subcutaneous, intradermal, intramuscular, intrapleural, topical, intralymphatic, or intracisternal administration; such introduction may also be performed intra-arterially, intracardially, subventricularly, epidurally, intracerebrally, intraventricularly, subretinally, intravitreally, intra-articularly, intraperitoneally, intrauterinely, or any combination thereof. In some embodiments, the delivery of the viral particles is systemic.

Administration of the AAV vectors, AAV particles, or compositions of the present disclosure can be achieved in one dose, continuously, or intermittently throughout the course of treatment. In some embodiments, the AAV vectors, AAV particles, or compositions of the present disclosure are administered parenterally by injection, infusion, or implantation. The AAV particles and compositions of the present disclosure can be administered in combination with other known treatments for the condition being treated.

### Reagent test kit

In some embodiments, the specific promoters, nucleic acid constructs, cells, viral particles or pharmaceutical compositions described herein can be assembled into pharmaceutical, or diagnostic or research kits, to facilitate their use in therapeutic, diagnostic or research applications. In some embodiments, the kits disclosed herein include any one of the AAV vectors, AAV particles, cells or pharmaceutical compositions described herein.

In some embodiments, the kit also includes instructions for use. Specifically, such a kit can include one or more reagents described herein, and instructions for describing the intended application and correct use of these reagents. In some embodiments, the kit can include instructions for one or more components of the mixed kit and/or separation and mixed samples and applied to the experimenter. In some embodiments, the reagent in the kit is a pharmaceutical preparation and dosage suitable for the method of administration of a specific application and reagent. Kits for research purposes can contain components of appropriate concentration or amount for carrying out various experiments.

The kit can be designed to facilitate the use of the methods described herein, and can take many forms. Where applicable, each composition of the kit can be provided in liquid form (e.g., in solution) or in solid form (e.g., dry powder). In some cases, some compositions can be configurable or otherwise processable (e.g., processed into an active form), for example, by adding a suitable solvent or other substance (e.g., water or cell culture medium), which may or may not be provided with the kit. In some embodiments, the composition can be provided in a preservation solution (e.g., a cryopreservation solution). The non-limiting example of a preservation solution includes DMSO, paraformaldehyde. In some embodiments, the preservation solution contains a certain amount of a metalloproteinase inhibitor.

In some embodiments, the test kit contains any one or more components described herein in one or more containers. Therefore, in some embodiments, the test kit can include a container for holding a reagent described herein. The reagent can be in the form of a liquid, gel or solid (powder). The reagent may be prepared under sterile conditions, packaged in syringes, and shipped under frozen storage. Alternatively, they can be contained in a vial or other container for storage. The second container can have other reagents prepared aseptically. Alternatively, the test kit can include a premixed active agent and transported in a syringe, vial, tube or other container. The test kit can have one or more or all components required for the reagent to be applied to the subject, such as a syringe, a local application device or an IV needle tube and a bag.

The present invention will be described below in the form of specific examples. It should be understood that these examples are merely illustrative and are not intended to limit the scope of the present description. Unless otherwise specified, the methods and materials used in the examples are conventional materials and methods in the art.

### EXAMPLE

### Example 1: Specific promoter promotes the expression of reporter gene in cochlear hair cells

### 1.1 Specific promoter Otov-HC Pasmid Construction and Viral Packaging

The specific promoter Otov-HC (SEQ ID NO:1) was inserted into the pAAV-mNeonGreen-Bpnls plasmid (flanked by AAV2 inverted terminal repeats) (SEQ ID NO:7). mNeonGreen (DNA sequence: SEQ ID NO:4, amino acid sequence: SEQ ID NO:5) is a green fluorescent protein used to indicate infected and expressing cells. The Otov-HC fragment (SEQ ID NO:1) was synthesized by GenScript and inserted upstream of the fluorescent protein mNeonGreen using the restriction enzymes Xbal and EcoRI, resulting in the plasmid pAAV-Otov-HC-mNeonGreen-Bpnls (SEQ ID NO:8). For viral packaging, this plasmid was co-transfected in appropriate amounts into HEK-293T cells along with the capsid protein fusion plasmid pAAV-ie-rep-cap (SEQ ID NO:10, provided by our laboratory( plasmid information can be found in doi: 10.1038/s41467-019-11687-8), and the pHelper plasmid (NCBI Reference: AF3699965.1). AAV virus was purified by iodixanol gradient centrifugation, and the virus titer was measured at 1E12-1E13 GC/mL.

### 1.2 Cochlear Hair Cell-Targeted Expression of mNeonGreen Driven by Specific Promoters

The above two AAV-ie:Otof-HC-mNeonGreen-Bpnls viruses containing specific promoters were injected into the inner ears of P2-P3 (postnatal days 2-3) mice via round window membrane injection. Mice were anesthetized on ice for 1-2 minutes. Following anesthesia, a postauricular incision was made to expose the round window niche, and the virus was manually injected through the round window membrane using a glass micropipette. The viral injection dose per ear was 6E10 GC. After injection, the skin incision was closed using 3M Vetbond tissue adhesive. The injected mice were then placed on a 37°C heating pad for recovery. Neonatal mice recovered completely within approximately 10 minutes and were subsequently returned to their mother. Standard postoperative care was administered. Two weeks later, the infection efficiency of cochlear hair cells was studied and statistically analyzed. Immunofluorescence staining was performed on mouse cochlea was performed using antibodies to the hair cell marker Myo7a, the supporting cell marker gene Sox2, and the nuclear marker DAPI. Before cochlear slabs were prepared, the cochlea was fixed and decalcified. After the cochlea was dissected and the tectorial membrane was removed, it was washed three times with PBS, blocked with Block solution for 1 hour, and then incubated with primary antibody (1:1000 Myo7a) at 4°C overnight. The tissues were washed three times with PBS and counterstained with secondary antibody (1:4000 Donkey anti-rabbit Alexa Fluor 555 and 1:1000 DAPI) at room temperature for 1 hour. And washed three times with PBS. The slide was removed, the mouse information and cochlear processing flow were marked, antiquenching agent was added to the slide, and the coverslip was gently covered to avoid air bubbles entering during the process. The slide was placed in a cool and dark place. Confocal images were taken on a Zeiss LSM700 laser confocal microscope. The complete cochlear image was processed using ImageJ software. The acquired images were projected along the Z-axis by ImageJ. Cells double-positive for Myo7a-labeled hair cells and mNeonGreen were counted. Immunofluorescence staining results showed that the mNeonGreen reporter gene was specifically expressed only in cochlear hair cells (Figure 2).

### Example 2: The Broad Promoter CAG (SEQ ID NO:6) Drives Widespread Expression of the Reporter Gene in Cochlear Cells

The pAAV-CAG-mNeonGreen-Bpnls plasmid was constructed and the virus was produced using the same method as in Example 1.

Using the same method as in Example 1, wild-type mice at P2 (postnatal day 2) underwent round window membrane virus injection. Immunofluorescence staining experiments were conducted at P14, with a virus injection dose of 6E10 GC per mouse ear. The results, as shown in Figure 1, demonstrate that AAV-ie-CAG-mNeonGreen-Bpnls can achieve 100% infection efficiency in cochlear hair cells of neonatal mice, and an infection efficiency of over 75% in supporting cells.

### Example 3: The SHH sequence (SEQ ID NO:3) alone does not drive gene expression in cochlear cells

### 3.1 Plasmid Construction and Virus Packaging

The SHH (SEQ ID NO:3) was constructed into the pAAV-mNeonGreen-Bpnls (SEQ ID NO:7) plasmid to construct the pAAV-SHH-mNeonGreen-Bpnls (SEQ ID NO:9) virus packaging plasmid. It was co-transfected with the AAV-ie capsid protein plasmid and the pHelper plasmid in appropriate amounts into HEK-293T cells. AAV virus was purified by iodide dialkyl gradient ultracentrifugation, and the virus titer was measured to be 1E+12 to 1E+13 GC/mL as the appropriate concentration.

### 3.2 SHH (SEQ ID NO:3) Alone Does Not Drive mNeonGreen Expression in Cochlear Cells

The above two AAV-ie: SHH-mNeonGreen-Bpnls viruses containing specific promoters were injected into the inner ears of P2-P3 (postnatal days 2-3) mice via round window membrane injection. Mice were anesthetized on ice for 1-2 minutes. Following anesthesia, a postauricular incision was made to expose the round window niche, and the virus was manually injected through the round window membrane using a glass micropipette. After injection, the skin incision was closed using 3M Vetbond tissue adhesive. Two weeks later, the infection efficiency of cochlear hair cells was studied and statistically analyzed. Immunofluorescence staining of mouse cochlea was performed with hair cell marker myosin7a and cell nucleus marker DAPI. Before cochlear slabs were prepared, the cochlea was fixed and decalcified. After the cochlea was dissected and the tectorial membrane was removed, it was washed three times with PBS, blocked with Block solution for 1 hour, and then incubated with primary antibody (1:1000 Myo7a) at 4°C overnight. The tissues were washed three times with PBS and counterstained with secondary antibody (1:4000 Donkey anti-rabbit Alexa Fluor 555 and 1:1000 DAPI) at room temperature for 1 hour. Cells double-positive for Myosin7a-labeled hair cells and mNeonGreen were counted. Immunofluorescence staining results showed that the mNeonGreen reporter gene was not expressed in cochlear hair cells and supporting cells (Figure 3). This indicates that Otov-HC (SEQ ID NO: 1) is specifically produced by SEQ ID NO: 2.

### Example 4: Targeted Driving Effect of the Specific Promoter on the Otof Gene (Dual Vectors) and Efficacy Verification

### 4.1 Plasmid Construction and Virus Packaging

Hearing loss mediated by the Otof gene is sensorineural non-syndromic deafness, which is expressed in the cochlear hair cells. Because the Otof gene is too large, it is necessary to use the double AAV vector pAAV-CMV-N-Otof-SD-AK plasmid (flanked by AAV2 inverted terminal repeats) (SEQ ID NO: 11) and pAAV-AK-SA-C-Otof plasmid (flanked by AAV2 inverted terminal repeats) (SEQ ID NO: 12) to deliver it to the inner ear hair cells. The specific promoter Otov-HC (SEQ ID NO: 1) was constructed into the pAAV-CMV-N-Otof-SD-AK plasmid (with AAV2 inverted terminal repeat sequences on both sides) (SEQ ID NO: 11). The plasmid construction was entrusted to GenScript to replace the original CMV promoter with the Otov-HC specific promoter (SEQ ID NO: 1) using the restriction enzymes Xbal and EcoRIto form the pAAV-Otov-HC-N-Otof-SD-AK virus packaging plasmid (SEQ ID NO: 13).which was co-transfected into HEK-293T cells with the pAAV-ie-rep-cap (SEQ ID NO: 10) capsid protein fusion plasmid provided by our laboratory (plasmid information can be found in the literature doi: 10.1038/s41467-019-11687-8.) and pHelper helper plasmid (NCBI No.: AF3699965.1) in appropriate amounts. AAV virus was purified by iodixanol gradient centrifugation, and the virus titer was measured at 1E12-1E13 GC/mL.

### 4.2 Efficacy Validation of the Otof Gene Knockout Mouse Model

The same method as in Example 1 was used to inject round window membrane virus into wild-type mice at P2 (postnatal day 2), and the virus injection volume per mouse ear was 6E10 GC. Auditory brainstem response (ABR) was used to detect the recovery of hearing function of Otof knockout mice after gene therapy at P30, and audiometry was performed on homozygous knockout mice 30 days after birth and wild-type control mice in the same litter. ABR is mainly used to detect whether the functions of all parts in the sound transmission process are normal. The sensory epithelium of the cochlea of WT, Otof-/- knockout mice and Otof-/- virus-injected mice after audiometry was microdissected and immunolabeled with Otoferlin (ab53233, 1:200, Abcam) and Alexa FluorTM 647 phalloidin antibody (Invitrogen, 1:100, A22287) to study the recombinant expression of the exogenous Otof gene driven by the specific promoter Otov-HC in the cochlear cells of KO mice (provided by Guangzhou Saiye Company).

The results showed that AAV-ie-Otov-HC-N-Otof-SD-AK and AAV-ie-AK-SA-C-Otof drove the specific expression of exogenous Otof gene in the cochlear hair cells of newborn mice, and the expression efficiency of recombinant Otof was close to that of WT mice in the same littermates, while homozygous knockout mice in the same littermates did not express the Otof gene (Figure 4). Statistical results showed that after 30 days, the expression efficiency of recombinant OTOF protein could still reach 100% in the 0-24kHz frequency band and more than 55% in the 24-32kHz frequency band (Figure 5). ABR results showed that after knocking out the Otof gene, the hearing threshold of mice increased significantly in low, medium and high frequencies, and they were basically completely deaf, indicating that Otof plays an important role in the hearing of mice. In the treated Otof knockout mouse model, 30 days after injection, the ABR threshold was lower than that of the homozygous knockout mice in the same litter, and the hearing frequency bands were significantly restored. The hearing recovery effect was similar to that of the same litter WT mice, and the hearing threshold remained stable for a considerable period of one month (Figure 6).

### The sequences of the present invention

SEQ ID NO: 1: Otov-HC promoter sequence
SEQ ID NO: 2: Enhancer sequence
SEQ ID NO: 3: SHH gene core promoter element sequence
SEQ ID NO: 4: mNeonGreen DNA sequence
SEQ ID NO: 5: mNeonGreen amino acid sequence
SEQ ID NO: 6: CAG sequence
SEQ ID NO: 7: pAAV-mNeonGreen-Bpnls plasmid sequence
SEQ ID NO: 8: pAAV-Otov-HC-mNeonGreen-Bpnls plasmid sequence
SEQ ID NO: 9: pAAV-SHH-mNeonGreen-Bpnls plasmid sequence
SEQ ID NO: 10: pAAV-ie-rep-cap capsid plasmid sequence
SEQ ID NO: 11: pAAV-promotor-CMV-N-Otof-SD-AK plasmid sequence
SEQ ID NO: 12: pAAV-AK-SA-C-Otof-WPRE-SV40 plasmid sequence
SEQ ID NO: 13: pAAV-Otov-HC-N-Otof-SD-AK virus packaging plasmid sequence

## Claims

1. A nucleic acid promoter comprising: (1) an enhancer sequence and (2) a core promoter element, wherein the enhancer sequence has a sequence shown in SEQ ID NO: 2 or a homologous sequence having at least 90% sequence identity thereto and retaining the function of SEQ ID NO: 2,
preferably, the homologous sequence is a sequence of mammals, preferably primates, and more preferably hominids.

2. The nucleic acid promoter according to claim 1, **characterized in that** the core promoter element described in (2) is the core promoter element of the SHH gene,
preferably, the core promoter element described in (2) comprises the sequence shown in SEQ ID NO: 3 or a homologous sequence having at least 90% sequence identity thereto,
more preferably, the homologous sequence is a sequence of mammals, preferably primates, and more preferably hominids.

3. The nucleic acid promoter according to claim 2, **characterized in that** the promoter comprises the sequence shown in SEQ ID NO: 1 or a sequence having at least 90% sequence identity thereto.

4. A nucleic acid construct comprising the promoter according to any one of claims 1 to 3,
preferably,
the nucleic acid construct is a vector, and/or
the nucleic acid construct further comprises a gene of interest, which is operably linked to the promoter,
more preferably, the gene is selected from: a deafness gene, a tool gene for manipulating genes of cochlear hair cells or expressed in cochlear hair cells.

5. An AAV vector system, comprising the nucleic acid construct of claim 4 and a nucleic acid construct encoding a gene required by the AAV virus,
preferably,
the nucleic acid construct further comprises a gene of interest, which is operably linked to the specific promoter,
the genes required for AAV virus packaging include one or more genes selected from the group consisting of: Rep, Cap, E2A, E4 and VA genes,
more preferably, the AAV vector system comprises an AAV-ie plasmid, a pHelper plasmid and the nucleic acid construct of claim 4.

6. An AAV virus particle comprising the promoter according to any one of claims 1 to 3.

7. A host cell comprising or having integrated into its genome: the promoter according to any one of claims 1 to 3, the nucleic acid construct according to claim 4, or the AAV vector system according to claim 5,
preferably, the cells are cochlear hair cells.

8. A pharmaceutical composition comprising a pharmaceutically acceptable excipient and one or more selected from the group consisting of: the promoter according to any one of claims 1 to 3, the nucleic acid construct according to claim 4, the AAV vector system according to claim 5, the AAV virus particle according to claim 6, and the host cell according to claim 7.

9. An in vitro method for expressing a gene in cochlear hair cells, comprising the steps of incubating the host cell according to claim 7 under conditions suitable for gene expression,
preferably, the method further comprises the step of introducing a nucleic acid construct comprising the gene into cochlear hair cells, wherein the gene is operably linked to the specific promoter according to any one of claims 1 to 3.

10. Use of the sequence shown in SEQ ID NO: 2 or a homologous sequence having at least 90% sequence identity thereto and retaining the function of SEQ ID NO: 2 in a chimeric promoter.

11. A method for preparing a chimeric promoter comprises: operably linking (1) a sequence shown in SEQ ID NO: 2 or a homologous sequence having at least 90% sequence identity thereto and retaining the function of SEQ ID NO: 2 with (2) a core promoter element.

12. Use of the promoter according to any one of claims 1 to 3 in the preparation of a drug for treating a disease caused by a gene defect in cochlear hair cells, wherein the drug is used to express the gene in cochlear hair cells,
preferably, the gene is selected from: a deafness gene, a tool gene for manipulating genes of cochlear hair cells or expressed in cochlear hair cells,
preferably, the disease is hereditary deafness caused by a gene defect in cochlear hair cells,
more preferably, the disease is sensorineural hearing loss.

13. A method for treating a disease caused by a gene defect in cochlear hair cells, comprising the step of expressing the gene in the cochlear hair cells of a subject, wherein the gene is operably linked to the promoter according to any one of claims 1 to 3,
preferably, the method comprises the step of introducing a nucleic acid construct or vector comprising the coding sequence of the gene into the cochlear hair cells, wherein the coding sequence of the gene is operably linked to the specific promoter,
preferably, the disease is hereditary deafness caused by a gene defect in cochlear hair cells,
more preferably, the disease is sensorineural hearing loss.
